# EUROPÄISCHE PATENTSCHRIFT

(11) **EP 3 099 403 B1**
(45) Veröffentlichungstag und Bekanntmachung des Hinweises auf die Patenterteilung: **17.03.2021**
(21) Anmeldenummer: 14825087.1
(22) Anmeldetag: 02.12.2014
(51) Int. Cl.: B01F 11/00

(54) **VORRICHTUNG UND VERFAHREN ZUM DURCHMISCHEN EINER MASSE**
DEVICE AND METHOD FOR INTERMIXING A COMPOUND
DISPOSITIF ET PROCÉDÉ DE MÉLANGEAGE D'UNE MASSE

(30) Priorität: 27.01.2014 AT 500482014
(43) Veröffentlichungstag der Anmeldung: 07.12.2016
(73) Patentinhaber: Fleck, Vinzenz, 8130 Frohnleiten (AT)
(72) Erfinder: Fleck, Vinzenz, 8130 Frohnleiten (AT)
(74) Vertreter: Wirnsberger, Gernot
(86) Internationale Anmeldenummer: PCT/AT2014/050290
(87) Internationale Veröffentlichungsnummer: WO 2015/109347

(56) Entgegenhaltungen:
- EP-A1- 1 533 024
- EP-A2- 0 196 291
- DE-A1- 3 512 548
- DE-A1- 3 512 548
- DE-T2-602004 004 649
- GB-A- 237 660
- GB-A- 237 660
- GB-A- 510 498
- GB-A- 2 145 634
- SU-A1- 877 833
- SU-A1- 877 833
- SU-A1- 1 134 227
- SU-A1- 1 134 227
- SU-A1- 1 592 026
- SU-A1- 1 592 026
- US-A- 3 164 303
- US-A- 3 164 303
- US-A- 4 511 254
- US-A- 4 946 286
- US-A1- 2009 027 997
- US-B1- 7 905 654

## Beschreibung

Die Erfindung betrifft eine Vorrichtung zum Durchmischen einer Masse wie einer Dispersion, umfassend einen Behälter, insbesondere einen großvolumigen Behälter, und eine am Behälter angeordnete Mischeinheit.

Weiter betrifft die Erfindung eine Verwendung einer Vorrichtung der vorstehend genannten Art.

Des Weiteren betrifft die Erfindung ein Verfahren zum Durchmischen einer Masse wie einer Dispersion in einem Behälter, insbesondere einem großvolumigen Behälter, mit einer am Behälter angeordneten Mischeinheit durch Bewegung derselben.

Ein Durchmischen einer Masse kann sowohl ein Homogenisieren oder ein Umwälzen einer heterogenen Masse als auch beispielsweise ein Rühren einer bereits homogenen Masse betreffen. Typische Durchmischprozesse finden unter anderem bei einer Herstellung von Gütern des täglichen Lebens Anwendung. Beispielsweise sind Misch- bzw. Durchmischprozesse bei einer Herstellung von diversen Kosmetika oder auch bei der Lebensmittelherstellung, z. B. bei der Behandlung von Maischen, erforderlich.

Aus dem Stand der Technik sind verschiedene Vorrichtungen und Verfahren zum Durchmischen einer Masse bekannt. Bei gängigen Vorrichtungen sind ein oder mehrere Propeller in einem Behälter angeordnet, wobei sich die zu durchmischende Masse ebenfalls im Behälter befindet. Durch eine hohe Drehzahl des Propellers wird versucht, die Masse zu durchmischen. In großvolumigen Behältern mit entsprechend großen Massen reicht dazu ein Propeller nicht aus; es ist eine Anordnung von mehreren Propellern notwendig, um ein vollständiges Durchmischen zu gewährleisten. Um die Propeller mit ausreichend hoher Drehzahl betreiben zu können, ist ein entsprechend großer Energieaufwand nötig. Weiter ist ein Zeitaufwand bis zum vollständigen Durchmischen einer Masse nicht zu unterschätzen. Bei heterogenen Massen, bei denen eine feste Phase überwiegt bzw. welche sehr dickflüssig sind, ist ein Durchmischen mittels Propeller manchmal nicht möglich. Die Propeller können in solch einer Masse bzw. hochviskosen Medien über kurz oder lang stecken bleiben bzw. werden diese geschädigt und sind nicht weiter verwendbar. Auch ist bei Massen in einem großvolumigen Behälter mit entsprechend großem Volumen ein vollständiges Durchmischen nicht möglich. Die Masse wird dabei nur lokal durchmischt bzw. diese wird vom Propeller nur bewegt, ohne dass eine Durchmischung erfolgt.

Weiter sind aus dem Stand der Technik Vorrichtungen zum Temperaturausgleich einer Masse in einem zumeist großvolumigen Behälter bekannt. Dabei wird die Masse bzw. Teile davon immer wieder bzw. dauerhaft umgepumpt. Dieser Prozess ist technisch aufwendig und kostenintensiv.

Eine weitere aus dem Stand der Technik bekannte Vorrichtung zum Durchmischen von Massen umfasst ein in einen Behälter einführbares, vertikal zu Behälterwänden angeordnetes Gitter. Ein solches Gitter wird vor allem bei der Herstellung von Rotwein verwendet. Die Abstände der einzelnen Gitterstäbe sind dabei relativ groß, sodass sich großflächige Löcher ergeben. Mit einer Vorrichtung dieser Art ist ein Durchmischen von heterogenen Massen oder ein Umwälzen einer großen Masse nicht zufriedenstellend möglich. Somit ist diese Art der Vorrichtung nur zur Rotweinherstellung verwendbar, allerdings nicht universell einsetzbar.

Aus dem Stand der Technik sind weiter verschiedene Mischvorrichtungen mit einer Kolben-Zylinder-Einheit bekannt, welche unterschiedliche Arten von Tauchsystemen in einen Behälter mit einer zu durchmischenden Masse eintauchen. Die dabei verwendeten Tauchelemente können verschiedenste Formen haben. Eine Art der Tauchelemente ist ähnlich einem Propeller ausgebildet und weist somit die oben diskutierten Nachteile auf. Andere Tauchelemente sind relativ klein ausgebildet und nahe am Kolben angeordnet. Diese Tauchelemente haben den Nachteil, dass es damit schwierig ist, eine Masse im Behälter vollständig zu durchmischen.

Mehrere Arten von Vorrichtungen zum Durchmischen einer Masse erfüllen deren Zweck für je eine bestimmte Art von Masse. Aus dem Stand der Technik ist jedoch keine Vorrichtung bekannt, welche sowohl möglichst viele heterogene als auch homogene Massen, letztere in Bezug auf einen Temperaturausgleich innerhalb der Masse, mit annähernd gleich gutem Resultat durchmischen bzw. auf eine bestimmte Temperatur bringen kann.

DE 35 12 548 A1 offenbart eine Vorrichtung entsprechend dem Oberbegriff des Anspruchs 1 und ein Verfahren entsprechend dem Oberbegriff des Anspruchs 12.

Aufgabe der Erfindung ist es demnach, eine Vorrichtung der eingangs genannten Art anzugeben, mit welcher ein einfaches und effizientes Durchmischen einer Masse möglich ist und welche möglichst universell einsetzbar ist.

Eine weitere Aufgabe der Erfindung ist es, eine Verwendung einer solchen Vorrichtung anzugeben.

Weiter ist es auch Aufgabe der Erfindung, ein Verfahren der eingangs genannten Art anzugeben, mit welchem eine Masse einfach und effizient durchmischt wird und welches beim Durchmischen von verschiedenen Arten von Massen eingesetzt werden kann.

Die erste Aufgabe wird durch eine Vorrichtung entsprechend Anspruch 1 gelöst.

Ein mit der Erfindung erzielter Vorteil ist insbesondere darin zu sehen, dass bei der linearen Bewegung des Kolbens ein Kraft- bzw. Energieverbrauch geringer als bei einer rotierenden Bewegung eines Propellers oder dergleichen ist. Die lineare Bewegung des Kolbens mit dem Mischelement führt dazu, dass sich die zu durchmischende Masse mit lokal unterschiedlichen Strömungsgeschwindigkeiten bewegt. Die Saugwirkung stellt in weiterer Folge wieder ein Kräftegleichgewicht her und die Masse wird somit durchmischt. Das flächige Mischelement bewirkt also sowohl ein vollständiges als auch ein schnelles Durchmischen der Masse. Eine Geschwindigkeit der linearen Bewegung ist dabei beliebig einstellbar, abhängig davon, welche Art von Masse zu durchmischen ist. Durch die flächige Erstreckung des Mischelementes um den Kolben ist eine große Umwälzung und damit ein rasches Durchmischen gewährleistet. Auch bei z. B. dickflüssigen Massen besteht kein Problem einer schnellen Abnutzung des Mischelementes, da keine Gefahr besteht, dass das flächige Mischelement in der Masse stecken bleibt. Weiter ermöglicht die erfindungsgemäße Vorrichtung ein Durchmischen von Massen mit unterschiedlichen Viskositäten, da die Saugwirkung bei allen Viskositäten von Massen auftritt. Darüber hinaus erlaubt es die Vorrichtung für eine homogene Masse eine bestimmte Temperatur einzustellen. Die unterschiedlichen lokalen Strömungsgeschwindigkeiten haben nicht nur Druckdifferenzen, sondern auch Temperaturdifferenzen zur Folge, welche ebenfalls die Kraft der Saugwirkung ausgleicht. Ein Ausgleich von Temperaturdifferenzen wirkt auch einem Entmischen einer Masse entgegen, was insbesondere in großvolumigen Behältern des Öfteren auftritt. Die erfindungsgemäße Vorrichtung ist insbesondere zum Durchmischen einer fließfähigen Masse geeignet.

Eine erfindungsgemäße Vorrichtung kann in allen Bereichen Anwendung finden, in denen in einem Behälter größeren Volumens von insbesondere mehr als fünf Litern eine Masse in Bezug auf einzelne Komponenten und/oder eine ausgeglichene Temperatur zu homogenisieren ist. Als besonders effektiv erweist sich eine erfindungsgemäße Vorrichtung vor allem bei der Umwälzung von Maische bei der Bierherstellung, insbesondere Starkbieren, die eine besonders dicke bzw. viskose Maische als Grundlage benötigen. Weiter erweist sich eine erfindungsgemäße Vorrichtung als geeignet, um Massen mit verschiedenen pH-Werten zu homogenisieren, z. B. bei Säureeintrag.

Bevorzugt ist vorgesehen, dass das Mischelement an einem ersten Ende des Kolbens gelagert ist. Dadurch wird erreicht, dass das Mischelement von einem Ende bis zu einem gegenüberliegenden Ende des Behälters bewegbar ist. Das erste Ende des Kolbens befindet sich dabei im Behälter, wohingegen ein zweites Ende des Kolbens in der Regel während eines gesamten Hubs außerhalb des Behälters bleibt und mit einem Antrieb in Verbindung steht. Vorteilhaft ist weiter, wenn das Mischelement während eines Durchmischprozesses stets unter einem Füllstandsniveau bleibt, um einen Lufteintrag möglichst gering zu halten.

Vorteilhaft ist es, wenn der Kolben um eine Längsachse drehbar gelagert ist, um ein noch schnelleres bzw. effektiveres Durchmischen einer Masse zu gewährleisten. Weiter ist durch diese zweite Möglichkeit einer Bewegung des Kolbens mit dem Mischelement ein noch breiterer Anwendungsbereich geschaffen. Insbesondere ermöglicht dies ein sehr individuelles Anpassen an verschiedene Arten von Massen bzw. es ist dieselbe Vorrichtung für verschiedene Arten von Massen verwendbar, wobei durch Aktivierung einer gleichzeitigen Drehbewegung während eines Hubs Mischprozesse optimierbar sind. Zusätzlich ist durch die Drehbewegung eine Stabilität des Kolbens mit dem Mischelement während einer linearen Bewegung erhöht.

Es ist von Vorteil, wenn der Kolben etwa in einer geometrischen Mitte des Mischelementes mit diesem verbunden ist, wobei sich das Mischelement etwa in einer Ebene um den Kolben erstreckt. Die mittige Anordnung des Mischelementes sorgt für ein vollständiges und gleichmäßiges Durchmischen der Masse.

Erfindungsgemäss ist vorgesehen, dass das Mischelement Aussparungen aufweist. Die Aussparungen in Kombination mit der linearen Bewegung des Kolbens und des Mischelementes bewirken ein noch effizienteres und schnelleres Durchmischen einer Masse. Eine Saugwirkung kann dabei beim linearen Bewegen des Kolbens in beide Richtungen auftreten. Eine überwiegende Wirkung beim Durchmischprozess erfolgt allerdings beim Herausziehen des Kolbens mit dem Mischelement. Durch die Aussparungen im Mischelement entstehen beim Bewegen desselben mehr bzw. feiner unterteilte lokale Strömungsgeschwindigkeiten. Dadurch ist also sichergestellt, dass die gesamte Masse nach einem Durchmischprozess vollständig durchmischt bzw. auf dieselbe Temperatur gebracht ist. Eine Geometrie der Aussparungen sowie deren Anzahl sind dabei frei wählbar, jedoch von einer Dicke bzw. Höhe des Mischelementes abhängig. Je dünner bzw. flacher ein Mischelement ausgebildet ist, desto weniger bzw. kleinere Aussparungen können vorgesehen sein. Weiter sind die Abmessungen des Mischelementes von einer zu durchmischenden Masse bzw. von einer Viskosität und einer Temperatur der Masse sowie einer Geschwindigkeit der linearen Bewegung des Kolbens abhängig. Darüber hinaus erfolgt eine Bewegung bzw. eine Durchmischung der Masse in Abhängigkeit der verschiedenen Parameter, welche sich wiederum gegenseitig beeinflussen.

Es kann auch vorgesehen sein, dass das Mischelement an einer flächigen Seite kegelförmige und/oder zylindrische Erweiterungen aufweist. Die kegelförmigen Erweiterungen unterstützen ein Durchmischen bei einer linearen Bewegung des Kolbens. Die zylindrischen Erweiterungen, welche sich vom Mischelement ausgehend gegen eine Rotationsrichtung des Kolbens erstrecken, sorgen für eine leichte Rotation desselben, um Turbulenzen im Behälter besser zu bewältigen. Es kann vorgesehen sein, dass die Erweiterungen an einem Mischelement mit Aussparungen angeordnet sind. Die kegelförmigen Erweiterungen können dabei flexibel bzw. biegbar ausgebildet sein, sodass diese je nach Bewegungsrichtung des Kolbens deren Position ändern.

Erfindungsgemäss ist vorgesehen, dass das Mischelement gewölbt ausgebildet ist. Dabei kann die Wölbung des Mischelementes in die zwei unterschiedlichen linearen Bewegungsrichtungen des Kolbens ausgebildet sein, abhängig von einer Art des Durchmischprozesses. Je nachdem welche Art von Masse durchmischt werden soll, ist das Mischelement entweder flach oder gewölbt ausgebildet. Möglich ist auch, dass das Mischelement flexibel ausgebildet ist und dadurch je nach Bewegungsrichtung des Kolbens bei einer Bewegungsrichtung in eine und bei einer gegensätzlichen Bewegungsrichtung in eine gegensätzliche Richtung gewölbt ist.

Der Kolben kann an beliebigen Positionen am Behälter gelagert sein. Möglich ist es, dass der Kolben horizontal beweg- bzw. verfahrbar ist. Üblicherweise ist der Kolben aber vertikal in einem Behälter bewegbar gelagert. In diesem Fall tritt die zu durchmischende Masse mit der Lagerstelle des Kolbens nicht in Berührung, sodass keine Sondermaßnahmen zur Abdichtung des Behälters vorzusehen sind.

Weiter ist es aus den gleichen Gründen von Vorteil, wenn die Mischeinheit an einem oberen Ende des Behälters fixiert ist. Bei dieser Anordnung muss der Behälter nicht gezwungenermaßen zur Mischeinheit hin vollständig abgedichtet sein. Es kann jedoch auch vorgesehen sein, dass der Behälter druckdicht verschließbar oder verschlossen ist.

Zweckmäßig ist es, wenn ein Verhältnis eines Durchmessers von Behälter zu Mischelement zumindest 1,2:1 ist. Vorgesehen ist, dass das Mischelement von einer Behälterwand beabstandet angeordnet ist. Der Durchmesser des Mischelementes beträgt zwischen 10 % und 40% des Durchmessers des Bodens des Behälters. Dadurch ist gewährleistet, dass die zum Durchmischen aufzuwendende Kraft möglichst klein, der Erfolg des Durchmischens jedoch möglichst groß ist. Es wird vermutet, dass bei einer Bewegung des Mischelementes nach oben Masse durch die Aussparungen im Mischelement in Richtung Boden fließt. Der so entstehende Druckunterschied ist dadurch ausgleichbar, dass Masse zwischen Mischelement und Wänden des Behälters nach oben fließt und die Masse somit durchmischt wird. Erfindungsgemäß kann vorgesehen sein, dass eine Geometrie des Mischelementes in Draufsicht einem Querschnitt des Behälters quer zur Bewegungsachse des Kolbens entspricht. Zum Beispiel ist bei einem im Querschnitt runden Behälter mit Vorteil ein in Draufsicht rundes Mischelement vorgesehen. Genaue Fließrichtungen und Verwirbelungen der Masse im Behälter bei einer Bewegung des Mischelementes sind wiederum von verschiedenen Parametern abhängig, wie von der Dicke und dem Durchmesser des Mischelementes, der Größe und der Anordnung der Aussparungen im Mischelement, der Viskosität und der Temperatur der Masse und der Geschwindigkeit und Art der Bewegung des Mischelementes.

Es kann vorgesehen sein, dass zumindest ein zusätzliches vom Kolben beabstandetes Rührwerk vorgesehen ist. Solch ein Rührwerk kann z. B. ein aus dem Stand der Technik bekannter Propeller sein. Dieser Propeller unterstützt das Mischelement bei aufwendig zu durchmischenden Massen. Der Propeller, welcher üblicherweise am Boden des Behälters angeordnet ist, hebt dabei die Masse vom Boden an und das Mischelement führt die restliche Durchmischung durch.

Bei besonders großvolumigen Behältern kann vorgesehen sein, dass zumindest ein zusätzliches Mischelement angeordnet ist, wobei das zusätzliche Mischelement vom ersten Mischelement beabstandet am Kolben fixiert ist. Durch das zusätzliche Mischelement ist eine Strecke der vertikalen Bewegung des Kolbens reduziert und infolgedessen eine Zeitdauer und ein Energieaufwand eines Durchmischprozesses einer Masse herabgesetzt, was schlussendlich auch eine Kostenreduktion zur Folge hat.

Eine Verwendung einer erfindungsgemäßen Vorrichtung eignet sich wie erläutert beim Herstellen von Maische.

Die verfahrensmäßige Aufgabe wird erfindungsgemäß durch ein Verfahren entsprechend Anspruch 12 gelöst.

Ein Vorteil des erfindungsgemäßen Verfahrens ist insbesondere darin zu sehen, dass durch das flächige Mischelement und die lineare Bewegung sehr viel Masse auf einmal durchmischt werden kann und dadurch in weiterer Folge eine Mischzeit und eine aufzuwendende Energie reduziert wird. Durch die lineare Bewegung des Kolbens mit dem Mischelement werden unterschiedliche lokale Strömungsgeschwindigkeiten und dadurch unterschiedliche Drücke und Temperaturen in der Masse eingestellt. Dieses Kräfteungleichgewicht wird durch eine Saugwirkung ausgeglichen und die Masse wird durchmischt bzw. auf eine für die gesamte Masse gleiche Temperatur gebracht. Weiter werden durch die lineare Bewegung Scherkräfte, welche die Masse schädigen können, minimal gehalten. Im Gegensatz zum Durchmischen mittels eines oder mehrerer Propeller kann eine Masse mit einer geringeren Anzahl von Bewegungsschritten durchmischt werden. Bevorzugt kann vorgesehen sein, dass die Mischeinheit mit einer Begrenzungsfläche des Behälters verbunden wird und das Mischelement dauerhaft im Behälter bewegbar gelagert wird. Durch diese Maßnahme wird die gesamte Vorrichtung nach außen abgegrenzt und das Verfahren kann ohne Massenverlust durch z. B. Überschwappen durchgeführt werden. Durch die dauerhafte Lagerung des Mischelementes im Behälter kommt dieses kaum in Kontakt mit Umgebungsluft und wird damit vor Korrosion oder dergleichen geschützt. Besonders bevorzugt ist vorgesehen, dass das Mischelement dauerhaft von der zu durchmischenden Masse bedeckt wird, um einen Lufteintrag minimal zu halten.

Für eine effektive Umwälzung ist vorgesehen, dass die Mischeinheit Aussparungen aufweist und die Masse unter Durchfließen durch die Aussparungen umgewälzt wird. Insbesondere bei Maischen bei der Alkoholherstellung, wo sich Feststoffe an einem Behälterboden absetzen, hat sich dies als vorteilhafte Variante erwiesen. Durch Heben des Kolbens bei vertikaler Lagerung desselben werden die Feststoffe vom Boden mit angehoben und müssen dann durch die Aussparungen nach unten austreten, was eine Verwirbelung ergibt und damit die gewünschte Durchmischung fördert.

Es kann bevorzugt vorgesehen sein, dass der Kolben mit dem Mischelement um eine Längsachse des Kolbens gedreht wird und dadurch die lineare Bewegung des Kolbens unterstützt wird. In weiterer Folge wird dadurch eine Durchmischzeit reduziert bzw. die Effektivität eines Durchmischprozesses nochmals gesteigert. Weiter wird dadurch eine Stabilität des Kolbens und des Mischelementes erreicht.

Weitere Merkmale, Vorteile und Wirkungen der Erfindung ergeben sich aus den dargestellten Ausführungsbeispielen. In den Zeichnungen, auf welche dabei Bezug genommen wird, zeigen:
Fig. 1 eine Vorrichtung;
Fig. 2 ein Teil einer Variante einer Vorrichtung in Draufsicht;
Fig. 3 der Teil der Variante der Vorrichtung gemäß Fig. 2 in einem Querschnitt gemäß der Linie III-III in Fig. 2;
Fig. 4 ein Teil einer weiteren Variante einer Vorrichtung;
Fig. 5 ein Teil einer erfindungsgemäßen Vorrichtung;
Fig. 6 ein Teil einer Variante einer erfindungsgemäßen Vorrichtung;
Fig. 7 ein Teil einer weiteren Variante einer Vorrichtung;
Fig. 8 ein Verfahren.

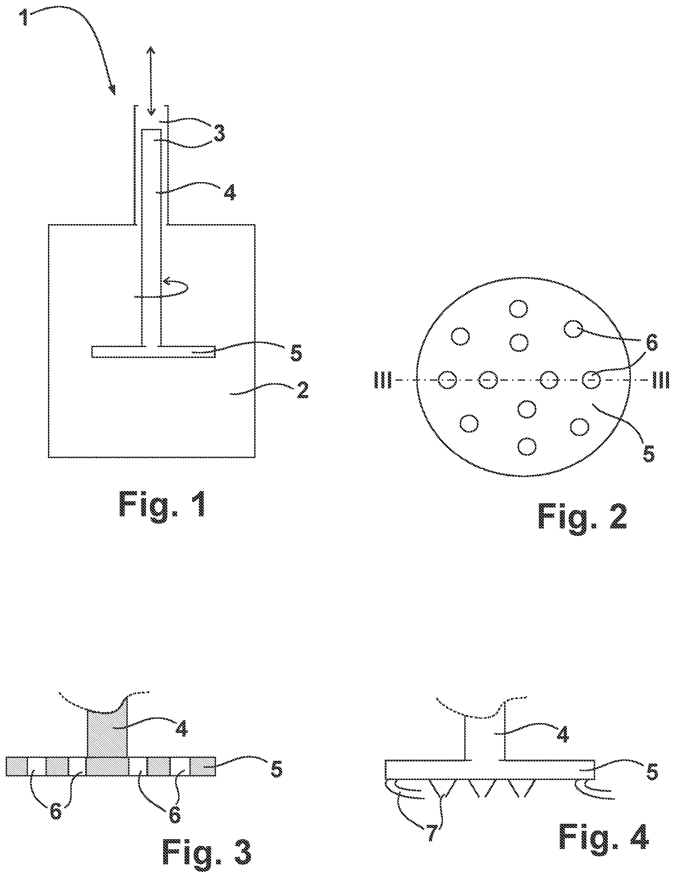

Fig. 1 zeigt eine Vorrichtung 1 zum Durchmischen einer Masse. Die Vorrichtung 1 umfasst einen Behälter 2 und eine Mischeinheit 3. Die Mischeinheit 3 ist dabei am Behälter 2 angeordnet und umfasst zumindest einen Kolben 4 mit einem Mischelement 5. Vorzugsweise ist die Mischeinheit 3 wie in Fig. 1 an einer oberen Seite des Behälters 2 angeordnet, es kann jedoch auch vorgesehen sein, dass diese an einer Seitenwand oder am Boden des Behälters 2 angeordnet ist. Weiter ist vorgesehen, dass der Kolben 4 mit dem Mischelement 5 im Behälter 2 gelagert ist. Der Behälter 2 mit der Mischeinheit 3 kann verschlossen oder mit einem Deckel oder einem anderen Verschluss verschließbar ausgebildet sein, um ein Austreten der Masse zu verhindern. Es kann auch vorgesehen sein, dass der Behälter 2 druckdicht verschließbar ist. Weiter ist vorgesehen, dass der Kolben 4 in zwei entgegengesetzte Richtungen linear bewegbar ist. Dadurch ist auch das am Kolben 4 angeordnete Mischelement 5 linear bewegbar. Die lineare Bewegung kann dabei beliebig angetrieben sein. Vorstellbar ist z. B. ein mechanischer, ein hydraulischer, ein elektrischer oder ein händischer Antrieb mittels Seilzug. Eine weitere Möglichkeit, die lineare Bewegung anzutreiben, kann eine Feder sein. Wenn der Kolben 4 mit dem Mischelement 5 an einer oberen Seite des Behälters 2 angeordnet ist, kann vorgesehen sein, dass dieser an einer Feder angeordnet ist, welche bei einer Bewegung des Kolbens 4 nach unten gespannt und bei einer Aufwärtsbewegung desselben losgelassen wird. Eine Geschwindigkeit der Bewegung ist der zu durchmischenden Masse anpassbar und bewegt sich üblicherweise zwischen 0,01 m/s und 10 m/s.

Das Mischelement 5 erstreckt sich flächig um den Kolben 4 und kann an einer beliebigen Stelle des Kolbens 4 angeordnet sein. Bevorzugt ist jedoch vorgesehen, dass das Mischelement 5 an einem Ende des Kolbens 4 gelagert ist und der Kolben 4 zum Optimieren einer Stabilität etwa mittig am Mischelement 5 fixiert ist. Das Mischelement 5 ist dadurch von einem ersten Ende des Behälters 2 zu einem zweiten Ende des Behälters 2 linear bewegbar, um die Masse vollständig zu durchmischen. Es kann vorgesehen sein, dass das Mischelement 5 lösbar am Kolben 4 gelagert ist. Dadurch ist das Mischelement 5 je nach Anforderung austauschbar. Zusätzlich zur linearen Bewegung kann erfindungsgemäß vorgesehen sein, dass der Kolben 4 mit dem Mischelement 5 um eine Längsachse des Kolbens 4 drehbar gelagert ist. Mit diesen zwei Bewegungsmöglichkeiten des Kolbens 4 und des Mischelementes 5 ist ein effektives und zeitsparendes Durchmischen der Masse gewährleistet. Gleichzeitig ist eine Stabilität des Kolbens 4 mit dem Mischelement 5 erhöht. Für die vollständige Durchmischung der Masse ist auch die flächige Erstreckung des Mischelementes 5 maßgeblich. Flächig ist hier als wesentlich zweidimensional zu verstehen bzw. es ist das Mischelement 5 wesentlich breiter als hoch bzw. dick, wobei eine breite Seite etwa senkrecht zum Kolben 4 angeordnet ist. Eine geometrische Form des Mischelementes 5 ist wiederum beliebig wählbar, jedoch ist diese auch davon abhängig, welche Masse durchmischt werden soll. Günstig ist es jedoch, wenn eine Geometrie des Mischelementes 5 in Draufsicht einem Querschnitt des Behälters quer zur Bewegungsachse des Kolbens entspricht. Zum Beispiel ist bei einem im Querschnitt runden Behälter mit Vorteil ein in Draufsicht rundes Mischelement vorgesehen. Andere erfindungsgemäße Formen des Mischelementes 5 können z. B. quadratisch, mehreckig oder wellenförmig sein. Günstig dabei ist, dass das Mischelement 5 von einer Wand des Behälters 2 beabstandet angeordnet ist.

Fig. 2 zeigt das Mischelement 5 in einer Variante. In dieser Variante ist das Mischelement 5 etwa rund ausgebildet und weist mehrere Aussparungen 6 auf. Die Aussparungen 6 können eine beliebige geometrische Form und Größe haben. In Fig. 2 sind die Aussparungen 6 als etwa runde Aussparungen 6 ausgeführt. Weiter können beliebig viele Aussparungen 6 vorgesehen sein, abhängig davon, welche Art von Masse durchmischt werden soll bzw. wie hoch eine Viskosität der Masse ist. Eine Voraussetzung hierfür ist, dass das Mischelement 5 dabei stets stabil bleibt und langlebig ist. Es hat sich gezeigt, dass das Mischelement 5 eine gewisse Mindestdicke aufweisen sollte, wenn heterogene Massen wirksam durchmischt werden sollten. Fig. 3 zeigt das Mischelement 5 gemäß Fig. 2 in einem Querschnitt gemäß der Linie III-III in Fig. 2.

Fig. 4 zeigt eine weitere Variante der erfindungsgemäßen Vorrichtung 1. Das Mischelement 5 weist hier an einer Seite kegelförmige und zylindrisch gebogene Erweiterungen 7 auf. In Fig. 4 sind die zylindrisch gebogenen Erweiterungen 7 an den Außenseiten angeordnet. Die Biegung der Erweiterungen 7 erstreckt sich gegen die Drehrichtung des Kolbens 4 um dessen Längsachse und sorgt für eine leichtere Rotation des Mischelementes 5. Erfindungsgemäß können die Erweiterungen 7 an beliebigen Stellen des Mischelementes 5 angeordnet sein. Weiter mittig in Fig. 4 sind drei kegelförmige Erweiterungen 7 am Mischelement 5 angeordnet. Die kegelförmigen Erweiterungen 7 unterstützen die lineare Bewegung des Kolbens 4 mit dem Mischelement 5 und verjüngen sich vom Mischelement 5 weg. Die kegelförmigen Erweiterungen 7 können auch flexibel bzw. umklappbar ausgebildet sein. Erfindungsgemäß kann vorgesehen sein, dass beide Arten der Erweiterungen 7 zusammen, wie in Fig. 4 dargestellt, oder je nur eine Art von Erweiterungen 7 an einem Mischelement 5 angeordnet sind. Erfindungsgemäß kann auch vorgesehen sein, dass die Erweiterungen 7 auf das Mischelement 5 aufgeschweißt sind. Es sind jedoch auch andere Herstellungsmöglichkeiten denkbar, z. B. eine einteilige Fertigung durch Stanzen und Biegung aus einem Blech.

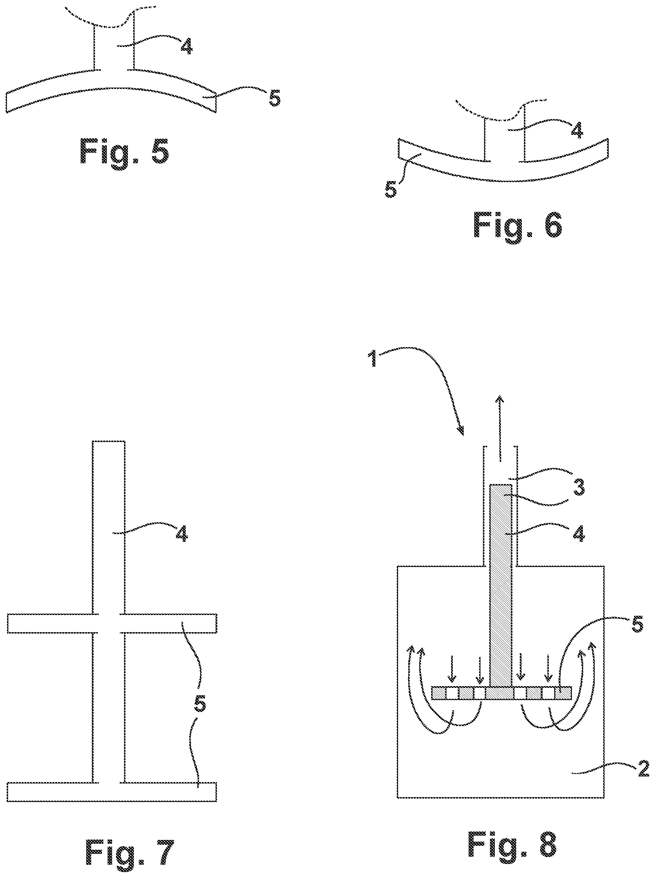

Fig. 5 und 6 zeigen zwei Varianten der erfindungsgemäßen Vorrichtung 1. Das Mischelement 5 ist in diesen Fällen nicht flach, sondern gewölbt ausgebildet. Die Wölbung kann dabei in beide Richtungen der linearen Bewegung des Kolbens 4 ausgebildet sein. Ist das Mischelement 5 an einem unteren Ende des Kolbens 4 angeordnet, kann die Wölbung gemäß Fig. 5 vom Kolben 4 weg oder gemäß Fig. 6 zum Kolben 4 hin ausgebildet sein. Eine Stärke der Wölbung ist beliebig wählbar. Weiter kann vorgesehen sein, dass das Mischelement 5 aus einem biegsamen Material ausgebildet ist und sich die Wölbung des Mischelementes 5, abhängig von dessen Bewegung, während des Durchmischprozesses ändert. Es kann auch vorgesehen sein, dass die Wölbung des Mischelementes 5 nicht gleichmäßig verläuft, sondern zusätzliche Biegungen aufweist.

Fig. 7 zeigt eine Vorrichtung 1 mit einem zusätzlichen Mischelement 5. Das zusätzliche Mischelement 5 ist vom ersten Mischelement 5 vertikal beabstandet am Kolben 4 angeordnet und weist dieselbe Form wie das erste Mischelement 5 auf.

Anwendung findet der Kolben 4 mit zwei oder mehreren daran angeordneten Mischelementen 5 beim Durchmischen von Massen in hohen Behältern 2. Es können beliebig viele Mischelemente 5 am Kolben 4 angeordnet sein und diese können jeweils andere Formen aufweisen. Zweckmäßig ist es, einen gewissen Abstand zwischen den einzelnen Mischelementen 5 einzuhalten, um Ablagerungen aus Teilen der Masse an diesen zu verhindern. Weiter sollten die Mischelemente 5 Aussparungen 6 aufweisen, um ein komplettes Durchmischen der Masse zu gewährleisten.

Der Behälter 2 kann z. B. ein Silo, ein Tank oder ein Schmelzgefäß sein. Dabei kann dieser zusätzlich mit Wärmeaustauschflächen ausgebildet sein, um einen Wärmeaustausch der Masse zu beschleunigen. Wenngleich die genannten Arten von Behältern 2 alle ein relativ großes Fassungsvermögen haben, ist die erfindungsgemäße Vorrichtung 1 bereits für Behälter 2 ab einem Fassungsvermögen von etwa fünf Litern verwendbar. Die Mischeinheit 3, insbesondere das Mischelement 5 und der Kolben 4, können aus verschiedensten Materialien hergestellt sein, z. B. aus Stahl, Edelstahl, Metall oder auch aus Beton, Kunststoff, Holz, Keramik oder Glas. Die verwendete Materialwahl muss auf Bedingungen der zu durchmischenden Masse angepasst sein. Gegebenenfalls kann das Mischelement 5 feuerfest ausgebildet sein, z. B. für Anwendungen in der Metall erzeugenden Industrie. Materialien zur Herstellung des Behälters 2 sind aus dem Stand der Technik bekannt. Optional kann eine Dichtung zwischen dem Behälter 2 und der Mischeinheit 3 vorgesehen sein, je nachdem, wo am Behälter 2 die Mischeinheit 3 angeordnet ist. Dabei kann es sich z. B. um eine statische, eine translatorische oder eine dynamische Dichtung handeln. Eine Steuerung der Vorrichtung kann manuell, elektrisch oder mechanisch, wie über eine Feder, erfolgen und ist individuell anpassbar.

Gegenwärtig ist noch nicht abschließend geklärt, wie das Durchmischen der Masse im Behälter 2 exakt erfolgt. In Fig. 8 ist der zurzeit vermutete Wirkungsmechanismus eines Verfahrens zum Durchmischen einer Masse, insbesondere einer fließfähigen Masse wie feinkörniges Pulver oder einer Dispersion, dargestellt. In Fig. 8 wird der Kolben 4 nach oben bewegt, also aus der Vorrichtung 1 raus, und gleichzeitig um eine Längsachse gedreht. Der Effekt des Durchmischens einer Masse tritt überwiegend beim Herausziehen des Kolbens 4 mit dem Mischelement 5 auf. Die Masse wird besonders effektiv durchmischt, wenn das Mischelement 5 mehrere Aussparungen 6 aufweist, wie in Fig. 8 dargestellt. Bei der Bewegung des Kolbens 4 mit dem Mischelement 5 nach oben wird die Masse durch die Aussparungen 6 im Mischelement 5 nach unten gedrückt. Um so entstehende Druckunterschiede auszugleichen, strömt Masse zwischen dem Mischelement 5 und den Wänden des Behälters 2 nach oben. Es kommt zu Verwirbelungen bzw. unterschiedlichen Strömungen und die Masse wird durchmischt. Der Prozess der Durchmischung bzw. die Bewegungsrichtungen der Masse ist in Fig. 8 durch Pfeile dargestellt. Vorteilhaft ist es, wenn das Mischelement 5 stets vollständig von der Masse bedeckt bleibt, um einen Lufteintrag möglichst gering zu halten. Es kann auch vorgesehen sein, dass zur Unterstützung des Mischelementes 5 am Boden des Behälters 2 ein Propeller angeordnet wird. Durch die lineare Bewegung des Kolbens 4 und gegebenenfalls zusätzliche Rotation desselben und des am Kolben 4 angeordneten Mischelementes 5 kann eine Masse relativ einfach, zeitsparend und energiesparend durchmischt werden. Eine Masse kann auch auf Temperatur gebracht, pH-homogenisiert oder beständig gelagert werden. Vorteilhaft ist weiter, dass Scherkräfte beim Durchmischprozess mit einer erfindungsgemäßen Vorrichtung 1 minimal gehalten werden. Dadurch kann die Vorrichtung 1 auch in Kombination mit Lagerbehältern verwendet werden.

Die Vorrichtung 1 zum Durchmischen von Massen kann universell eingesetzt werden; es können Massen mit Viskositäten im Bereich von 1 mPas bis zu 500 Pas durchmischt werden. Somit können mit der Vorrichtung 1 z. B. Dispersionen, Suspensionen, Maischen, Schlämme, Abwasser, fluidisierte Stoffgemenge und Schmelzen durchmischt und/oder geheizt und/oder gekühlt und/oder pH-homogenisiert werden. Eine Ausführungsform des Mischelementes 5 muss dabei auf die Viskosität und die Temperatur der zu durchmischenden Masse angepasst werden. Eine erfindungsgemäße Vorrichtung 1 findet auch in Gärtanks Anwendung. Insbesondere in großvolumigen Gärtanks findet oftmals eine Entmischung der Masse durch Temperaturdifferenzen statt. Diesem unerwünschten Prozess kann mit der Vorrichtung 1 entgegengewirkt werden. Dadurch ergibt sich eine Anwendbarkeit in verschiedensten Bereichen wie z. B. in der Lebensmittelindustrie, in der Chemie, in der Baustofftechnik, in der Kosmetik, in der Pharmaproduktion, in der Biotechnologie, in der Brauerei, in der Brennerei, in der Gießerei oder in der Tank-Logistik. Insbesondere für eine Anwendung in der Gießerei sollte das Mischelement 5 feuerfest ausgebildet sein. Die Mischeinheit 3 kann mit jeder Art eines Behälters 2 verwendet werden, gegebenenfalls kann diese auch nachträglich an einem Behälter 2 angeordnet werden. Weiter kann diese auch unterstützend eingesetzt werden, z. B. in Kombination mit einem aus dem Stand der Technik bekannten Propeller. Es kann vorgesehen sein, dass die Mischeinheit 5 lösbar am Kolben 4 angeordnet ist, um für verschiedene Massen ausgetauscht werden zu können. Dazu kann ein Stutzen an einem oberen Ende des Mischelementes 5 vorgesehen sein, in welchen der Kolben 4 hineingesteckt und befestigt werden kann. Alternativ kann der Stutzen auch am Kolben 4 angeordnet sein, um mit dem Mischelement 5 in Verbindung zu treten.

## Patentansprüche

1. Vorrichtung (1) zum Durchmischen einer Masse wie einer Dispersion, umfassend einen großvolumigen Behälter (2) mit einer Behälterwand und eine am Behälter (2) angeordnete Mischeinheit (3), wobei die Mischeinheit (3) zumindest einen im Behälter (2) gelagerten, linear bewegbaren Kolben (4) mit zumindest einem Mischelement (5) umfasst und der Behälter (2) einen Boden mit einem Durchmesser umfasst, wobei sich das Mischelement (5) flächig um den Kolben (4) erstreckt, um die Masse zu durchmischen, wobei das Mischelement (5) Aussparungen (6) aufweist und von der Behälterwand beabstandet angeordnet ist, wobei das Mischelement (5) gewölbt ausgebildet ist, **dadurch gekennzeichnet, dass** ein Durchmesser des Mischelementes (5) 10 % bis 40 % des Durchmessers des Bodens beträgt.

2. Vorrichtung (1) nach Anspruch 1, **dadurch gekennzeichnet, dass** das Mischelement (5) an einem ersten Ende des Kolbens (4) gelagert ist.

3. Vorrichtung (1) nach Anspruch 1 oder 2, **dadurch gekennzeichnet, dass** der Kolben (4) um eine Längsachse drehbar gelagert ist.

4. Vorrichtung (1) nach einem der Ansprüche 1 bis 3, **dadurch gekennzeichnet, dass** der Kolben (4) etwa in einer geometrischen Mitte des Mischelementes (5) mit diesem verbunden ist, wobei sich das Mischelement (5) etwa in einer Ebene um den Kolben (4) erstreckt.

5. Vorrichtung (1) nach einem der Ansprüche 1 bis 4, **dadurch gekennzeichnet, dass** das Mischelement (5) an einer flächigen Seite kegelförmige und/oder zylindrisch gebogene Erweiterungen (7) aufweist.

6. Vorrichtung (1) nach einem der Ansprüche 1 bis 5, **dadurch gekennzeichnet, dass** der Kolben (4) vertikal bewegbar gelagert ist.

7. Vorrichtung (1) nach Anspruch 6, **dadurch gekennzeichnet, dass** die Mischeinheit (3) an einem oberen Ende des Behälters (2) fixiert ist.

8. Vorrichtung (1) nach einem der Ansprüche 1 bis 7, **dadurch gekennzeichnet, dass** ein Verhältnis eines Durchmessers von Behälter (2) zu Mischelement (5) zumindest 1,2:1 beträgt.

9. Vorrichtung (1) nach einem der Ansprüche 1 bis 8, **dadurch gekennzeichnet, dass** zumindest ein zusätzliches vom Kolben (4) beabstandetes Rührwerk vorgesehen ist.

10. Vorrichtung (1) nach einem der Ansprüche 1 bis 9 **dadurch gekennzeichnet, dass** zumindest ein zusätzliches Mischelement (5) angeordnet ist, wobei das zusätzliche Mischelement (5) vom ersten Mischelement (5) beabstandet am Kolben (4) fixiert ist.

11. Verwendung einer Vorrichtung (1) nach einem der Ansprüche 1 bis 10 zum Herstellen von Maische.

12. Verfahren zum Durchmischen einer Masse wie einer Dispersion in einem großvolumigen Behälter (2) mit einem Boden mit einem Durchmesser, mit einer am Behälter (2) angeordneten Mischeinheit (3) durch Bewegung derselben, wobei die Mischeinheit (3) zumindest einen Kolben (4) und zumindest ein Mischelement (5) mit Aussparungen (6) umfasst, wobei das Mischelement (5) flächig um den Kolben (4) gelagert ist, und der Kolben (4) linear bewegt wird, um die Masse zu durchmischen, wobei das Mischelement (5) gewölbt ausgebildet ist und von einer Behälterwand beabstandet angeordnet ist, wobei die Masse unter Durchfließen durch die Aussparungen (6) und Strömen durch einen Abstand zwischen einer Behälterwand und Mischelement (5) umgewälzt wird, **dadurch gekennzeichnet, dass** das Mischelement (5) einen Durchmesser von 10 % bis 40 % des Durchmessers des Bodens aufweist.

13. Verfahren nach Anspruch 12, **dadurch gekennzeichnet, dass** der Kolben (4) mit dem Mischelement (5) um eine Längsachse des Kolbens (4) gedreht wird.

## Claims

1. Apparatus (1) for intermixing a mass such as a dispersion, comprising a large-volume container (2) with a container wall, and a mixing unit (3) arranged on the container (2), wherein the mixing unit (3) comprises at least one linearly displaceable piston (4) mounted in said container (2) and has at least one mixing element (5), and said container comprises a bottom with a diameter, wherein the mixing element (5) extends two-dimensionally around the piston (4) for the purpose of intermixing the mass, wherein the mixing element (5) has recesses (6) and is arranged at a distance from the container wall, wherein the mixing element (5) is of convex construction, **characterized in that** a diameter of the mixing element (5) is equal to 10 % to 40 % of the bottom.

2. Apparatus (1) according to claim 1, **characterized in that** the mixing element (5) is mounted on a first end of the piston (4).

3. Apparatus (1) according to claim 1 or 2, **characterized in that** the piston (4) is mounted so as to be rotatable about a longitudinal axis.

4. Apparatus (1) according to any one of claims 1 to 3, **characterized in that** the piston (4) is connected to said apparatus approximately in a geometric centre of the mixing element (5), wherein the mixing element (5) extends approximately in a plane about the piston (4).

5. Apparatus (1) according to any one of claims 1 to 4, **characterized in that** the mixing element (5) has conical and/or cylindrically curved extensions (7) on a flat side thereof.

6. Apparatus (1) according to any one of claims 1 to 5, **characterized in that** the piston (4) is mounted so as to be movable vertically.

7. Apparatus (1) according to claim 6, **characterized in that** the mixing unit (3) is fixed to an upper end of the container (2).

8. Apparatus (1) according to any one of claims 1 to 7, **characterized in that** a ratio between a diameter of the container (2) and the mixing element (5) is at least 1.2:1.

9. Apparatus (1) according to any one of claims 1 to 8, **characterized in that** at least one additional agitator is provided, located at a distance from the piston (4).

10. Apparatus (1) according to any one of claims 1 to 9, **characterized in that** at least one additional mixing element (5) is arranged, wherein the additional mixing element (5) from the first mixing element (5) is fixed at a distance from the piston (4).

11. Use of an apparatus (1) according to any one of claims 1 to 10 for making mash.

12. Method for intermixing a mass such as a dispersion in a large-volume container (2) with a bottom having a diameter, with a mixing unit (3) arranged on the container (2), by movement thereof, wherein the mixing unit (3) comprises at least one piston (4) and at least one mixing element (5) with recesses (6), wherein the mixing element (5) is mounted two-dimensionally around the piston (4) and the piston (4) is moved linearly in order to intermix the mass, wherein the mixing element (5) is of convex construction and is arranged at a distance from a container wall, wherein the mass is circulated while flowing through the recesses and passing through a gap between a container wall and mixing element (5), **characterized in that** the mixing element (5) has a diameter equal to 10 % to 40 % of the diameter of the bottom.

13. Method according to claim 12, **characterized in that** the piston (4) is rotated with the mixing element (5) about a longitudinal axis of the piston (4).

## Revendications

1. Dispositif (1) de mélange d'une masse telle qu'une dispersion, comprenant un récipient de grand volume (2) avec une paroi de récipient et un ensemble mélangeur (3) disposé sur le récipient (2), sachant que l'ensemble mélangeur (3) comprend au moins un piston (4) supporté dans le récipient (2), pouvant se déplacer linéairement avec au moins un ensemble mélangeur (5) et le récipient (2) comprend un fond avec un diamètre, sachant que l'élément mélangeur (5) s'étend à plat autour du piston (4) pour mélanger la masse, sachant que l'élément mélangeur (5) comporte des évidements (6) et est disposé distant de la paroi de récipient, sachant que l'élément mélangeur (5) est constitué bombé, **caractérisé en ce qu'**un diamètre de l'élément mélangeur (5) est 10 % à 40 % du diamètre du fond.

2. Dispositif (1) selon la revendication 1, **caractérisé en ce que** l'élément mélangeur (5) est supporté à une première extrémité du piston (4).

3. Dispositif (1) selon la revendication 1 ou 2, **caractérisé en ce que** le piston (4) est supporté pouvant tourner autour d'un axe longitudinal.

4. Dispositif (1) selon l'une quelconque des revendications 1 à 3, **caractérisé en ce que** le piston (4) est relié à celui-ci à peu près dans un centre géométrique de l'élément mélangeur (5), sachant que l'élément mélangeur (5) s'étend à peu près dans un plan autour du piston (4).

5. Dispositif (1) selon l'une quelconque des revendications 1 à 4, **caractérisé en ce que** l'élément mélangeur (5) comporte sur sa face plane des élargissements (7) coniques et/ou courbés de façon cylindrique.

6. Dispositif (1) selon l'une quelconque des revendications 1 à 5, **caractérisé en ce que** le piston (4) est supporté pouvant se déplacer verticalement.

7. Dispositif (1) selon la revendication 6, **caractérisé en ce que** l'ensemble mélangeur (3) est fixé à une extrémité supérieure du récipient (2).

8. Dispositif (1) selon l'une quelconque des revendications 1 à 7, **caractérisé en ce qu'**un rapport entre un diamètre du récipient (2) et l'ensemble mélangeur (5) est au moins de 1,2:1.

9. Dispositif (1) selon l'une quelconque des revendications 1 à 8, **caractérisé en ce qu'**au moins un agitateur supplémentaire distant du piston (4) est prévu.

10. Dispositif (1) selon l'une quelconque des revendications 1 à 9, **caractérisé en ce qu'**au moins un élément mélangeur supplémentaire (5) est disposé, sachant que l'élément mélangeur supplémentaire (5) est fixé sur le piston (4) distant du premier élément mélangeur (5).

11. Utilisation d'un dispositif (1) selon l'une quelconque des revendications 1 à 10 pour la fabrication d'un moût.

12. Procédé de mélange d'une masse telle qu'une dispersion dans un récipient de grand volume (2) avec un fond avec un diamètre, avec un ensemble mélangeur (3) disposé sur le récipient (2) par mouvement de celle-ci, sachant que l'ensemble mélangeur (3) comprend au moins un piston (4) et au moins un élément mélangeur (5) avec des évidements (6), sachant que l'élément mélangeur (5) est supporté à plat autour du piston (4) et le piston (4) est déplacé linéairement pour mélanger la masse, sachant que l'élément mélangeur (5) est constitué bombé et est disposé distant d'une paroi de récipient, sachant que la masse est mise en circulation par écoulement à travers les évidements (6) et par écoulement à travers un espace entre une paroi de récipient et un élément mélangeur (5), **caractérisé en ce que** l'élément mélangeur (5) comporte un diamètre de 10 % à 40 % du diamètre du fond.

13. Procédé selon la revendication 12, **caractérisé en ce que** le piston (4) est tourné avec l'élément mélangeur (5) autour d'un axe longitudinal du piston (4).
